Europäisches Patentamt

European Patent Office

Office européen des brevets

(11) Veröffentlichungsnummer: **0 411 315 A1**

## (12) EUROPÄISCHE PATENTANMELDUNG

(21) Anmeldenummer: 90112198.8

(22) Anmeldetag: 27.06.90

(51) Int. Cl.⁵: **A61K 31/44**, //(A61K31/44, 31:135)

(30) Priorität: 02.08.89 DE 3925540

(43) Veröffentlichungstag der Anmeldung:
06.02.91 Patentblatt 91/06

(84) Benannte Vertragsstaaten:
AT BE CH ES FR GB GR IT LI LU NL SE

(71) Anmelder: SCHÜLKE & MAYR GMBHMBH
Heidbergstrasse 100
D-2000 Norderstedt(DE)

(72) Erfinder: Eggensperger, Heinz, Dr.
Alsterallee 13
D-2000 Hamburg 65(DE)
Erfinder: Goroncy-Bermes, Peter, Dr.
Woldenhornstieg 19
D-2070 Ahrensburg(DE)
Erfinder: Harke, Hans-Peter, Dr.
Theodor-Fahr-Strasse 27
D-2000 Hamburg 62(DE)
Erfinder: Töfke, Susanne, Dr.
Rutschbahn 15
D-2000 Hamburg 13(DE)

(74) Vertreter: UEXKÜLL & STOLBERG
Patentanwältelte
Beselerstrasse 4
D-2000 Hamburg 52(DE)

(54) Wässrige antiseptische Zusammensetzung.

(57) Die Erfindung betrifft eine wäßrige antiseptische Zusammensetzung, die insbesondere als Schleimhautantiseptikum und zur Wundbehandlung geeignet ist und einen Gehalt an
a) N,N′-(1,10 Decandiyldi-1[4H]pyridinyl-4-yliden)bis-(1-octanamin)dihydrochlorid (Octenidin) und
b) Phenoxyethanol (POE und/oder Phenoxypropanol (POP) in einem Gewichtsverhältnis von Octenidin zu POE bzw. POP von 1:20 bis 1:1 bei einer Octenidin-Konzentration von 0,005 bis 0,5 Gew.% und einer POE- bzw. POP-Konzentration von 5 bis 0,5 Gew.% aufweist.

EP 0 411 315 A1

## WÄSSRIGE ANTISEPTISCHE ZUSAMMENSETZUNG

Die Erfindung betrifft eine wäßrige antiseptische Zusammensetzung, insbesondere als Schleimhautantiseptikum und zur Wundbehandlung.

Antimikrobiell wirkende wäßrige Antiseptika auf Basis von N,N'-(1,10 Decandiyldi-1[4H]pyridinyl-4-yliden)bis-(1-octanamin)dihydrochlorid sind aus der US-PS 4 442 215 bekannt. Diese als Octenidinhydrochlorid bezeichnete Verbindung wird in Wasser und gegebenenfalls in Mischungen mit Ethylalkohol, Isopropylalkohol, Propylenglykol und/oder Glycerin als Desinfektionsmittel eingesetzt. Es ist ferner bekannt, daß 0,1 g Octenidindihydrochlorid in einer 75%igen Lösung aus Propylalkohol und Isopropylalkohol als Hautdesinfektionsmittel geeignet ist, so z.B. H-P. Harke in Zbl.Hyg. 188, 189 bis 193 (1989).

Antiseptika werden vor operativen Eingriffen, vor Injektionen oder Punktionen und vor Inspektionen von Hohlorganen eingesetzt, wenn die Haut oder die Schleimhaut desinfiziert werden muß. Antiseptika werden auch zur Wundbehandlung, zur Behandlung lokaler oberflächlicher Infektionen eingesetzt, jedoch sind derartige antiseptische Lösungen mit einem hohen Alkoholgehalt an der Schleimhaut, an Übergangsepithelien und an verletzter Haut aus Gründen der Toxikologie und Akzeptans nicht einsetzbar.

Andere bislang bekannte Schleimhautantiseptika wie Polyvinylpyrrolidon/Jod, Phenylquecksilbersalze, Taurolin oder $H_2O_2$ sind aus toxikologischer oder ökologischer Sicht oder wegen ihrer geringen Wirksamkeit kaum geeignet.

Der Erfindung liegt die Aufgabe zugrunde, eine insbesondere für die Schleimhaut geeignete antiseptische Zusammensetzung vorzuschlagen, die nicht nur die sofortige Verminderung, sondern auch eine möglichst lang anhaltende Vermehrungshemmung von potentiellen Krankheitserregern auf der Schleimhaut ermöglicht und gleichzeitig die physiologische Flora schont, um neben einer einmaligen prophylaktischen Antiseptik eine lang dauernde regelmäßige prophylaktische Antiseptik und auch eine therapeutische Antiseptik zu ermöglichen.

Zur Lösung dieser Aufgabe wird eine wäßrige antiseptische Zusammensetzung gemäß Patentanspruch vorgeschlagen.

Überraschenderweise zeigen die erfindungsgemäßen Antiseptika in der Kombination von Octenidin mit POE bzw. POP neben einem schnellen Wirkungseintritt und einer lang anhaltenden Wirkung an der Haut bzw. Schleimhaut einen Synergismus. An sich sind aromatisch substituierte Alkohole wie POE und POP in geringen Konzentrationen nicht sonderlich antimikrobiell wirksam; in Kombination mit Octenidin wird dagegen nicht nur überraschenderweise ein schneller Wirkungseintritt und zusätzlich eine Langzeitwirkung erreicht, sondern es wird sogar dann eine ausreichende Keimreduktion erzielt, wenn man bei einem gleichbleibenden Gehalt von beispielsweise 1% bis 2% POE bzw. POP die Octenidin-Konzentration bis auf 0,005 verringert.

Die Tatsache, daß durch die Anwesenheit von POE bzw. POP in Kombination mit Octenidin ein schneller Wirkungseintritt und gleichzeitig eine Langzeitwirkung bei der Schleimhautdesinfektion bewirken, und zwar mit sehr niedrigen Octenidin-Konzentrationen erfolgt, beruht nicht nur auf einer synergistischen oder überadditiven Wirkung, sondern vermutlich auf anderen bislang noch nicht geklärten Phänomenen in Zusammenhang mit einem intrazellularen Transport von Wirkstoffen.

Wesentlich ist im vorliegenden Fall die deutliche Reduktion der Einsatzkonzentrationen des Octenidins bei niedriger Einsatzkonzentration von POE bzw. POP, um wirksame und schmerzfreie Präparate mit guter lokaler Verträglichkeit zu erhalten. Darüber hinaus ergeben sich aus technischer und medizinischer Sicht erhebliche Vorteile, weil die Wirkstoffe in den notwendigen niedrigen Konzentrationen wasserlöslich sind und man auf Lösungsmittel oder Lösungsvermittler verzichten kann, die die toxikologischen Eigenschaften der Präparate nachteilig beeinflussen können. Hinzu kommt, daß die Kombination von Octenidin mit POE bzw. POP neben ökologischen und toxikologischen Vorteilen die lokale Verträglichkeit beim Einsatz der erfindungsgemäßen Antiseptika verbessert und insbesondere bei intakter oder geschädigter Haut keinen Schmerz verursacht.

Im folgenden wird die Erfindung anhand von Beispielen näher erläutert:

Beispiel 1:

Es wurden Octenidin, Phenoxyethanol (POE) und Phenoxypropanol (POP) alleine und in der erfindungsgemäßen Kombination von Octenidin/POE bzw. Octenidin/POP in wäßriger Lösung auf ihre mikrobizide Wirksamkeit unter Verwendung von Staph.aureus untersucht, wobei die Bestimmung der bakteriziden Wirkung nach den Richtlinien der Deutschen Gesellschaft für Hygiene und Mikrobiologie (DGHM) erfolgte.

2

Die Einwirkungszeiten im quantitativen Suspensionsversuch betrugen 5 bzw. 15 bzw. 30 Sekunden. Vergleichweise wurde eine 0,2%ige Octenidin-Lösung mit 10% n-Propylalkohol untersucht.

Phenoxyethanol (POE) sowie Phenoxypropanol (POP) und N-Propanol haben als Einzelwirkstoffe keine Wirkung; in der folgenden Tabelle I ist die mikrobizide Wirksamkeit von Octenidin allein bzw. mit + 2% POE bzw. 1,0% POP und als Vergleichmit 10% n-Propylalkohol wiedergegeben, und zwar als Wirksamkeit wäßriger Lösungen von 0,2% Octenidin, 0,2% Octenidin und 2 % Phenoxyethanol, 0,2 % Octenidin und 1 % Phenoxypropanol, 0,2 % Octenidin und 10 % n-Propanol sowie deren Verdünnungen mit Wasser gegen Staph. aureus innerhalb von 5, 15 oder 30 Sekunden im quantitativen Suspensionsversuch. Angegeben ist die log. Keimreduktion (= log. Keimzahl vor minus log. Keimzahl nach Einwirkung der jeweiligen Lösung auf die Keime).

Tabelle I

| OCTENIDIN 0,2 % | | 5″ | 15″ | 30″ |
|---|---|---|---|---|
| | 100 % | ≧ 5,34 | ≧ 5,41 | ≧ 5,32 |
| | 75 % | 2,62 | ≧ 5,41 | ≧ 5,32 |
| | 50 % | 2,95 | 4,13 | ≧ 5,32 |
| | 25 % | 0,69 | 1,24 | 1,93 |
| | 8 % | 0,65 | 1,26 | 1,51 |
| OCTENIDIN 0,2 % + 2,0 % POE | | | | |
| | 100 % | ≧ 5,34 | ≧ 5,41 | ≧ 5,23 |
| | 75 % | ≧ 5,34 | ≧ 5,41 | ≧ 5,23 |
| | 50 % | ≧ 5,34 | ≧ 5,41 | ≧ 5,23 |
| | 25 % | 3,54 | ≧ 5,41 | ≧ 5,23 |
| | 8 % | 1,29 | 1,82 | 2,17 |
| OCTENIDIN 0,2 % + 1 % POP | | | | |
| | 100 % | ≧ 5,34 | ≧ 5,41 | ≧ 5,23 |
| | 75 % | ≧ 5,34 | ≧ 5,41 | ≧ 5,23 |
| | 50 % | ≧ 5,34 | ≧ 5,41 | ≧ 5,23 |
| | 25 % | 2,92 | 3,47 | 3,52 |
| | 8 % | 1,08 | 1,71 | 3,62 |
| OCTENIDIN 0,2 % + 10 % n-Prop. | | | | |
| | 100 % | ≧ 5,34 | ≧ 5,41 | ≧ 5,23 |
| | 75 % | 3,91 | 4,61 | 4,05 |
| | 50 % | 3,59 | ≧ 5,41 | ≧ 5,23 |
| | 25 % | 2,67 | 2,68 | 4,62 |
| | 8 % | 1,19 | 1,43 | 1,64 |

Die obige Tabelle zeigt, daß die mikrobizide Wirksamkeit der erfindungsgemäßen Zusammensetzung erheblich besser ist.

Beispiel 2:

Es wurde ein qualitativer Suspensionsversuch mit verschiedenen Keimarten durchgeführt, und zwar einmal mit Octenidin als alleinigem Wirkstoff und zum anderen mit einer erfindungsgemäßen Kombination von Octenidin mit Phenoxyethanol (POE).

Die Werte sind in der folgenden Tabelle II aufgeführt und zeigen, daß die erfindungsgemäße Kombination von 0,025% Octenidin und 0,5% Phenoxyethanol im Suspensionsversuch mit verschiedenen Keimarten bei einer Einwirkungszeit von 5 Minuten sehr viel wirksamer ist als das antimikrobiell wirkende Octenidin ohne andere Zusätze.

EP 0 411 315 A1

## Tabelle II

| % Octenidin | Staph.aur. | E.coli | Prot.mirab. | Ps.aerug. | C.albicans |
|---|---|---|---|---|---|
| 0,1 | - | - | - | - | - |
| 0,05 | + | - | + | - | - |
| 0,025 | + | + | + | + | + |

| % OCTENIDIN | % HEROXYEHIANOL | Staph.aureus | E.coli | Prot.mirabilis | Ps.aeruginosa | C.albicans |
|---|---|---|---|---|---|---|
| 0,1 | 2 | - | - | - | - | - |
| 0,05 | 1 | - | - | - | - | - |
| 0,025 | 0,5 | - | - | + | - | - |

Beispiel 3:

Es wurde die Wirksamkeit von Octenidin gegen Staph.aureus in einem quantitativen Suspensionsversuch einmal ohne Zusatz und zum anderen mit einem Zusatz von 2% POE durchgeführt. Die 1g Reduktionsfaktoren (RF-Werte) wurden nach einer Einwirkungszeit von 5 bzw. 15 bzw. 30 bzw. 60 Sekunden ermittelt, wie die folgende Tabelle III a für Octenidin alleine und Tabelle III b für Octenidin mit 2% POE zeigen.

Tabelle III a

| OCTENIDIN | $5''$ | $15''$ | $30''$ |
|---|---|---|---|
| 0,2 % | ≧5,34 | ≧5,41 | ≧5,32 |
| 0,15 % | 2,62 | ≧5,41 | ≧5,32 |
| 0,1 % | 2,95 | 4,13 | ≧5,32 |
| 0,05 % | 0,69 | 1,24 | 1,93 |
| 0,016 % | 0,65 | 1,26 | 1,51 |

Tabelle III b

| | RF-Werte nach | | | |
|---|---|---|---|---|
| | $5''$ | $15''$ | $30''$ | $60''$ |
| | Einwirkungszeit | | | |
| 0,100 % Octenidin + 2 % POE | ≧4,63 | ≧5,40 | ≧5,91 | ≧5,49 |
| 0,050 % Octenidin + 2 % POE | ≧4,87 | ≧5,40 | ≧5,91 | ≧5,49 |
| 0,025 % Octenidin + 2 % POE | ≧5,54 | ≧5,40 | ≧5,91 | ≧5,49 |
| 0,0125 % Octenidin + 2 % POE | ≧4,75 | ≧4,76 | ≧5,37 | ≧5,56 |
| 0,006 % Octenidin + 2 % POE | ≧5,45 | ≧5,30 | ≧5,42 | ≧5,56 |

Die obige Tabelle IIIb zeigt den nicht nur durch Synergismus erklärbaren Effekt, daß unter Beibehaltung einer 2%igen POE-Kozentration der Anteil an Octenidin bis auf 0,006% abgesenkt werden kann, und dennoch die Keimzahlreduktion, unabhängig von der Einwirkungszeit, hervorragende Werte zeigt.

Beispiel 4:

Die gleiche Wirkung zeigt Phenoxypropanol (POP). Die Werte für die Keimzahlreduktion gegenüber Staph.aureus bei konstantem POP-Gehalt und sich verringernder Octenidin-Konzentration ergaben analog Beispiel 3 wiederum überraschenderweise nahezu gleiche RF-Werte bei konstantem Alkoholgehalt und Octenidin-Konzentrationen, die von 0,1 bis 0,006% verringert wurden, wie die folgende Tabelle IV zeigt.

Tabelle IV

| | RF - WERTE NACH | | | |
|---|---|---|---|---|
| | 5" | 15" | 30" | 60" EINWIRKUNGSZEIT |
| 0,100 % OCTENIDIN + 1 % POP | ≥ 5,65 | ≥ 5,32 | ≥ 6,11 | ≥ 5,61 |
| 0,050 % OCTENIDIN + 1 % POP | ≥ 5,65 | ≥ 5,32 | ≥ 6,11 | ≥ 5,61 |
| 0,025 % OCTENIDIN + 1 % POP | 5,05 | 5,02 | ≥ 6,11 | ≥ 5,61 |
| 0,0125% OCTENIDIN + 1 % POP | 3,83 | 5,02 | ≥ 6,11 | ≥ 5,61 |
| 0,006 % OCTENIDIN + 1 % POP | 5,05 | 4,72 | ≥ 6,11 | ≥ 5,61 |

Beispiel 5:

Es wurde die Reduktion der Keimzahlen nunmehr mit gram-negativen Bakterien, nämlich mit Pseudomonas aeruginosa einmal mittels Octenidin in verschiedenen Konzentrationen und zum anderen mit der erfindungsgemäßen Kombination mit POE bzw. POP bestimmt, wobei der Gehalt an aromatisch substituiertem Alkohol aliquot verringert wurde.

Diese Werte sind in der folgenden Tabelle V wiedergegeben und zeigen, daß die Reduktionsfaktoren der erfindungsgemäßen Kombination von Octenidin und POE bzw. mit POP auch bei sinkender Konzentration der aromatischen Alkohole erheblich besser ist als von Octenidin allein bzw. der aromatischen Alkohole allein.

TABELLE V

| WIRKSTOFF | REDUKTIONSFAKTOREN NACH | | | |
|---|---|---|---|---|
| | 5" | 15" | 30" | 60" EINWIRKUNGSZEIT |
| 0,15 % OCTENIDIN | 3,25 | 4,45 | ≥ 5,46 | ≥ 5,15 |
| 0,10 % OCTENIDIN | 2,21 | 3,54 | ≥ 5,46 | ≥ 5,15 |
| 0,05 % OCTENIDIN | 1,15 | 1,59 | 2,45 | 3,79 |
| 0,025% OCTENIDIN | 0,79 | 0,98 | 1,10 | 1,38 |
| 0,15 % OCTENIDIN + 1,5 % POE | ≥ 4,94 | ≥ 4,90 | ≥ 4,80 | ≥ 4,86 |
| 0,10 % OCTENIDIN + 1,0 % POE | ≥ 4,94 | ≥ 4,90 | ≥ 4,80 | ≥ 4,86 |
| 0,05 % OCTENIDIN + 0,5 % POE | 3,00 | ≥ 4,90 | ≥ 4,80 | ≥ 4,86 |
| 0,025% OCTENIDIN + 0,25% POE | 0,35 | 0,64 | 1,08 | 1,91 |
| 0,15 % OCTENIDIN + 0,75 % POP | 3,89 | ≥ 6,65 | ≥ 6,62 | ≥ 6,74 |
| 0,10 % OCTENIDIN + 0,50 % POP | 2,72 | 3,49 | 4,72 | ≥ 6,74 |
| 0,05 % OCTENIDIN + 0,25 % POP | 2,68 | 3,02 | 3,62 | ≥ 6,74 |
| 0,025% OCTENIDIN + 0,125% POP | 0,93 | 1,32 | 1,82 | 3,12 |
| 1,5 % PHENOXYETHANOL | 0 | 0 | 0 | 0,17 |
| 0,75 % PHENOXYPROPANOL | 0,07 | 0 | 0,04 | 0 |

Beispiel 6:

Es wurden analog Beispiel 5 die Reduktion der Keimzahlen durch Octenidin als Einzelwirkstoff bzw. in der erfindungsgemäßen Kombination mit den aromatischen Alkoholen POE bzw. POP gegenüber Staph.aureus bestimmt.

Die Werte sind in der folgenden Tabelle VI aufgeführt und zeigen wie die Werte gemäß Beispiel 5 eine erhebliche Wirkungssteigerung beim Einsatz von Octenidin mit POE bzw. POP, und zwar auch bei sinkender Konzentration der aromatischen Alkohole.

Tabelle VI

| WIRKSTOFF | REDUKTIONSFAKTOREN NACH | | |
|---|---|---|---|
| | $5''$ | $15''$ | $30''$ EINWIRKUNGSZEIT |
| 0,2 % OCTENIDIN | $\geq 5,34$ | $\geq 5,41$ | $\geq 5,32$ |
| 0,15 % OCTENIDIN | 3,06 | $\geq 5,41$ | $\geq 5,23$ |
| 0,10 % OCTENIDIN | 1,85 | 4,36 | $\geq 5,23$ |
| 0,05 % OCTENIDIN | 0,38 | 1,19 | 1,87 |
| 0,016 % OCTENIDIN | 0,32 | 0,76 | 1,12 |
| 0,2 % OCTENIDIN + 2,0 % POE | $\geq 5,34$ | $\geq 5,41$ | $\geq 5,23$ |
| 0,15 % OCTENIDIN + 1,5 % POE | $\geq 5,34$ | $\geq 5,41$ | $\geq 5,23$ |
| 0,10 % OCTENIDIN + 1,0 % POE | $\geq 5,34$ | $\geq 5,41$ | $\geq 5,23$ |
| 0,05 % OCTENIDIN + 0,5 % POE | 3,64 | $\geq 5,41$ | $\geq 5,23$ |
| 0,016 % OCTENIDIN + 0,16% POE | 1,30 | 1,56 | 1,79 |
| 0,2 % OCTENIDIN + 1,0 % POP | $\geq 5,34$ | $\geq 5,41$ | $\geq 5,23$ |
| 0,15 % OCTENIDIN + 0,75 % POP | $\geq 5,34$ | $\geq 5,41$ | $\geq 5,23$ |
| 0,10 % OCTENIDIN + 0,5 % POP | $\geq 5,53$ | $\geq 5,51$ | $\geq 5,57$ |
| 0,05 % OCTENIDIN + 0,25 % POP | 4,08 | $\geq 5,51$ | $\geq 5,57$ |
| 0,016 % OCTENIDIN + 0,08 % POP | 1,06 | 1,43 | 2,62 |
| 2 % PHENOXYETHANOL | 0 | 0,33 | 0,12 |
| 1 % PHENOXYPROPANOL | 0,02 | 0 | 0 |

Beispiel 7:

Es wurde die Wirksamkeit verschiedener Antiseptika in der Mundhöhle nach einer Anwendungszeit von 20 Sekunden hinsichtlich ihrer Sofortwirkung und hinsichtlich ihrer Remanenzwirkung untersucht. Hierbei wurden die Reduktionsfaktoren nach der Antiseptik bei einer erfindungsgemäßen Zusammensetzung aus 0,1% Octenidin und 2% POE bestimmt und mit anderen üblichen Antiseptika verglichen. Die Werte ergeben sich aus der folgenden Tabelle VII.

Tabelle VII zeigt, daß die erfindungsgemäße Zusammensetzung sowohl hinsichtlich der Sofortwirkung als auch hinsichtlich der Remanenzwirkung sehr viel bessere Werte zeigt.

TABELLE VII

| Zeitpunkt nach Antiseptik | 0,1 % Octenidin | H₂O | H₂O₂ | Chlorhexidin | PVP Jod | |
|---|---|---|---|---|---|---|
| | 2 % Phenoxyethanol | | 3% | 0,5 % | 1:8 | |
| 0 | - | 0.11 | 0.94 | - | - | Sofortwirkung |
| 1 | 2.00 | - | - | - | - | |
| 2 | - | - | - | - | - | |
| 5 | - | 0.21 | 0.39 | 1.42 | 0.85 | |
| 30 | 1.01 | 0.21 | - | 1.1 | 0.56 | Remanenzwirkung |
| 60 | 0.92 | - | 0.31 | - | - | |
| 120 | 0.76 | - | - | - | - | |

Beispiel 8:

Es wurde ein Schleimhautantiseptikum aus 0,1 Gew.% Octenidin und 2,0 Gew.% Phenoxyethanol (POE) in wäßriger Lösung hergestellt, welches ohne weitere Zusätze sowohl zur einmaligen prophylaktischen Antiseptik, zur Reduktion der gesamten Mikroflora für die Dauer des Eingriffs als auch für eine regelmäßige prophylaktische Antiseptik, nämlich für eine Reduktion der pathologischen Mikroflora bei weitgehender Schonung der natürlichen Mikroflora und letztlich auch zur terapheutischen Antiseptik bei selektiver Reduktion bzw. Eliminierung von Infektionserregern auf Schleimhaut bei weitgehender Schonung der physiologischen Mikroflora eingesetzt werden konnte.

Beispiel 9:

Es wurde eine Zusammensetzung gemäß Beispiel 9 hergestellt, wobei in einem Fall noch 1,0 Gew.% Kokosaminopropylbetain und in einem anderen Fall noch 0,4% Natriumgluconat und 0,5% Glycerin eingesetzt wurden.

Weitere Zusätze zu den erfindungsgemäßen Mischungen sind möglich, wie beispielsweise Tensideemulgatoren, Mittel zur Beeinflussung der Konsistenz, ölige Zusätze und Rückfettungsmittel, ferner Feuchthaltemittel und Lösungsvermittler sowie Puffersubstanzen, Schaumstabilisatoren oder Entschäumer, Parfums und Riechstoffe. Beispielsweise können die erfindungsgemäßen Produkte noch Natriumgluconat, Glycerin, Tylose, Fettsäuremonoglyceride, ethoxylierte Fettalkoholpolyglykolether, Fettalkohole, Silikonöl, Feuchthaltemittel enthalten.

Letztlich ist es auch möglich, die erfindungsgemäße Kombination aus Octenidin und Phenoxyethanol bzw. Phenoxypropanol zusammen mit einem Trägermaterial wie Maisstärke oder einem SiO₂-Xerogel als Tablette zu formulieren, die in Wasser aufgelöst eingesetzt werden kann.

Beispiel 11a bis c:

Es wurden die folgenden Zusammensetzungen mit POE hergestellt, die wahlweise auch mit POP oder einem Gemisch von POP und POE angesetzt werden können.

| Beispiel 11a | |
|---|---|
| Octenidindihydrochlorid | 0,1 % |
| Phenoxyethanol | 2,0 % |
| Cocosamidopropylbetain | 1,0 % |
| NaOH | 0,01-% |
| Natriumgluconat | 0,4 % |
| Glycerin | 0,5 % |
| Tylose VH 100 000 P | 2,5 % |
| Wasser | ad 100 % |

| Beispiel 11b | |
|---|---|
| Octenidindihydrochlorid | 0,1 % |
| Phenoxyethanol | 1,0 % |
| Fettsäuremonoglycerid | 5,0 % |
| ethoxylierter Fettalkoholpolyglykolether | 6,0 % |
| Fettalkohole | 16,0 % |
| Siliconöl | 0,5 % |
| Feuchthaltemittel | 4,0 % |
| Wasser | ad 100 % |

| Beispiel 11c | |
|---|---|
| Octenidindihydrochlorid | 0,1 % |
| Phenoxyethanol | 2,0 % |
| Polyoxyethylenfettsäuremonoglycerid | 12,0 % |
| Fettalkohole | 19,0 % |
| Siliconöl | 0,5 % |
| Feuchthaltemittel | 4,0 % |
| Wasser | ad 100 % |

**Ansprüche**

Wäßrige antiseptische Zusammensetzung, insbesondere als Schleimhautantiseptikum und zur Wundbehandlung, **gekennzeichnet** durch einen Gehalt an

a) N,N'-(1,10 Decandiyldi-1[4H]pyridinyl-4-yliden)bis-(1-octanamin)dihydrochlorid (Octenidin) und

b) Phenoxyethanol (POE) und/oder Phenoxypropanol (POP)

in einem Gewichtsverhältnis von Octenidin zu POE bzw. POP von 1:20 bis 1:1 bei einer Octenidin-Konzentration von 0,005 bis 0,5 Gew.% und einer POE- bzw. POP-Konzentration von 5 bis 0,5 Gew.%.

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| **EINSCHLÄGIGE DOKUMENTE** ||||
| A | "Rote Liste", 1989, Editio Cantor, Aulendorf/Württ., DE<br>* Nr. 32063: "Octeniderm" *<br>----- | 1 | A 61 K 31/44 //<br>(A 61 K 31/44<br>A 61 K 31:135) |
| | | | **RECHERCHIERTE SACHGEBIETE (Int. Cl.5)**<br><br>A 61 K |

Der vorliegende Recherchenbericht wurde für alle Patentansprüche erstellt

| Recherchenort | Abschlußdatum der Recherche | Prüfer |
|---|---|---|
| DEN HAAG | 10-10-1990 | BRINKMANN C. |